# EUROPÄISCHE PATENTSCHRIFT

(11) **EP 3 837 595 B1**
(45) Veröffentlichungstag und Bekanntmachung des Hinweises auf die Patenterteilung: **29.03.2023**
(21) Anmeldenummer: 19725976.5
(22) Anmeldetag: 21.05.2019
(51) Int. Cl.: G06F 3/01, G06F 3/0481, G06T 15/00, G06T 19/00, G02B 27/01, G06F 3/04815

(54) **STEUERVORRICHTUNG UND VERFAHREN ZUM REDUZIEREN VON BEWEGUNGSKRANKHEIT EINES BENUTZERS BEIM BETRACHTEN EINES MEDIENINHALTS MITTELS EINER DATENBRILLE WÄHREND EINER FAHRT IN EINEM KRAFTFAHRZEUG**
CONTROL APPARATUS AND METHOD FOR REDUCING MOTION SICKNESS IN A USER WHEN LOOKING AT A MEDIA CONTENT BY MEANS OF SMART GLASSES WHILE TRAVELLING IN A MOTOR VEHICLE
DISPOSITIF DE COMMANDE ET PROCÉDÉ DESTINÉ À RÉDUIRE LE MAL DES TRANSPORTS D'UN UTILISATEUR PAR VISUALISATION D'UN CONTENU MULTIMÉDIA AU MOYEN DE LUNETTES INTELLIGENTES PENDANT UN TRAJET DANS UN VÉHICULE AUTOMOBILE

(30) Priorität: 13.08.2018 DE 102018213588
(43) Veröffentlichungstag der Anmeldung: 23.06.2021
(73) Patentinhaber: Audi AG, 85045 Ingolstadt (DE)
(72) Erfinder: PROFENDINER, Daniel, 85049 Ingolstadt (DE)
(74) Vertreter: Hofstetter, Schurack & Partner
(86) Internationale Anmeldenummer: PCT/EP2019/063020
(87) Internationale Veröffentlichungsnummer: WO 2020/035185

(56) Entgegenhaltungen:
- WO-A1-2018/057980
- US-A1- 2015 097 863
- US-A1- 2018 081 426
- PHILIPP HOCK ET AL: "CarVR", PROCEEDINGS OF THE 2017 CHI CONFERENCE ON HUMAN FACTORS IN COMPUTING SYSTEMS , CHI '17, ACM PRESS, NEW YORK, NEW YORK, USA, 2. Mai 2017 (2017-05-02), Seiten 4034-4044, XP058337783, DOI: 10.1145/3025453.3025665 ISBN: 978-1-4503-4655-9

## Beschreibung

Die Erfindung betrifft ein Verfahren zum Reduzieren von Bewegungskrankheit (Motion-Sickness oder Kinetose), die ein Benutzer beim Betrachten eines Medieninhalts (zum Beispiel eines Unterhaltungsfilms) empfinden kann, wenn er während einer Fahrt mit einem Kraftfahrzeug für das Betrachten eine Datenbrille benutzt. Zu der Erfindung gehört auch eine Steuervorrichtung für eine Datenbrille eines Kraftfahrzeugs. Die Erfindung umfasst schließlich auch ein Kraftfahrzeug mit einer solchen Steuervorrichtung.

Bei einem System zur Nutzung der sogenannten virtuellen Realität (VR - Virtual Reality) in einem Kraftfahrzeug wird daran gearbeitet, dass neben vollimmersiven Spielen auch 2D-Medieninhalte (Filme, Text, Präsentationen, um nur Beispiele zu nennen) ohne das Empfinden von Bewegungskrankheit oder mit einer reduzierten Empfindung von Bewegungskrankheit genutzt werden können. Die besagte virtuelle Realität wird hierbei mittels einer Datenbrille vermittelt, welche die Augen des Benutzers zur Umgebung hin lichtdicht abschließt und im Blickfeld des Benutzers stattdessen mittels eines jeweiligen Bildschirms für jedes Auge ein virtueller Raum aus einer künstlichen Perspektive, d.h. von einem künstlichen Betrachtungspunkt aus, angezeigt wird. Die aus der Perspektive dargestellten Anzeigeinhalte der virtuellen Realität (d.h. des virtuellen Raums) werden dabei in Bezug auf eine Bewegung eines Kopfes des Benutzers angepasst, wodurch der Benutzer ein sogenanntes immersives Erlebnis empfindet, das heißt eine Korrelation zwischen einer Bewegung seines Kopfes und der sich hiermit verändernden Perspektive. Für den Benutzer ergibt sich dadurch ein bewegungskorrelierte Perspektive, wie sie sich auch ohne Datenbrille bei der Betrachtung der realen Umgebung ergeben würde.

Bei der Benutzung in einem Kraftfahrzeug ergeben sich aber zusätzliche Bewegungen des Kraftfahrzeugs selbst (translatorische Bewegung beim Fahren und Rotation z.B. bei Kurvenfahrt oder beim Nicken), was im Anzeigeinhalt der virtuellen Realität nicht unbedingt abgebildet sein muss, wenn der Benutzer währenddessen den Kopf bezüglich des Kraftfahrzeugs ruhig hält. Der Benutzer spürt dann eine Beschleunigungskraft der Fahrzeugbewegung, für die es keine visuelle Entsprechung in der virtuellen Realität gibt. Dies führt der besagten Bewegungskrankheit.

Aus der DE 10 2014 019 579 A1 ist bekannt, dass man mittels der Datenbrille den anzuzeigenden Medieninhalt teiltransparent darstellen kann und zusammen mit dem Medieninhalt mittels der teiltransparenten Darstellung auch einen künstlichen Hintergrund oder eine künstliche Umgebung darstellen kann, deren Objekte sich relativ zu der in der Datenbrille angezeigten Perspektive (das heißt dem virtuellen Standpunkt des Benutzers und seiner virtuellen Blickrichtung) entsprechend den Kraftfahrzeugbewegungen bewegen. Dies reduziert zwar die Bewegungskrankheit, ergibt aber im dargestellten oder angezeigten Medieninhalt Bewegungsfragmente des teiltransparent dargestellten Hintergrunds.

Aus der DE 101 56 219 C1 ist bekannt, mittels der Datenbrille den Medieninhalt nur in einem verkleinerten Medienfeld darzustellen, das heißt einem vorbestimmten Bildbereich, und dieses Medienfeld in Abhängigkeit von Signalen eines Bewegungs- und Positionssensors des Fahrzeugs zu kippen, um hierdurch dem Benutzer der Datenbrille die Bewegung des Kraftfahrzeugs bezüglich der Umgebung visuell zu vermitteln. Dies hat aber zur Folge, dass der Medieninhalt vor der Perspektive des Benutzers hin und her kippt.

Aus der WO 2018/057980 A1 ist bekannt, die Bewegungskrankheit dadurch zu reduzieren, dass ein künstlicher, ruhender Horizont eingeblendet wird und zusätzlich in einem peripheren Bereich des Blicks des Benutzers künstliche Umgebungsobjekte animiert werden, die sich gemäß der Bewegung des Kraftfahrzeugs am virtuellen Blickpunkt des Benutzers vorbeibewegen. Hierdurch können aber eine Beschleunigung und ein Abbremsen des Kraftfahrzeugs visuell nicht direkt vermittelt werden, sondern es ergibt sich nur indirekt eine schnellere oder langsamere Driftgeschwindigkeit der virtuellen Objekte. Aber gerade auf die visuelle Vermittlung von Beschleunigungskräften kommt es an.

Aus einer wissenschaftlichen Veröffentlichung von Hock et al. (Philipp Hock et al., "CarVR", PROCEEDINGS OF THE 2017 CHI CONFERENCE ON HUMAN FACTORS IN COMPUTING SYSTEMS, CHI '17, ACM PRESS, NEWYORK, NEW YORK, USA, 2. Mai 2017 (2017-05-02), Seiten 4034-4044, DOI: 10.1145/3025453.3025665, ISBN: 978-1-4503-4655-9) ist bekannt, als visuelles Orientierungselement zur Veranschaulichung von gemessenen Beschleunigungskräften einen Rahmen eines Cockpitfensters in eine VR-Anzeige zu integrieren.

Mit den aus dem Stand der Technik bekannten Lösungen kann somit der Haupteinfluss für die Bewegungskrankheit, nämlich das Wirken der Beschleunigungskräfte auf den Gleichgewichtssinn des Benutzers, nicht visuell korrespondierend vermittelt werden.

Der Erfindung liegt die Aufgabe zugrunde, bei der Benutzung einer Datenbrille während einer Fahrt eines Kraftfahrzeugs die Bewegungskrankheit des Benutzers beim Betrachten eines Medieninhalts zu reduzieren.

Die Aufgabe wird durch die Gegenstände der unabhängigen Patentansprüche gelöst. Vorteilhafte Ausführungsformen der Erfindung sind durch die abhängigen Patentansprüche, die folgende Beschreibung sowie die Figur beschrieben.

Durch die Erfindung ist ein Verfahren zum Reduzieren der Bewegungskrankheit eines Benutzers beim Betrachten eines Medieninhalts bereitgestellt. Das Verfahren geht davon aus, dass der Benutzer den Medieninhalt mittels einer Datenbrille während einer Fahrt in einem Kraftfahrzeug betrachtet. Es werden in an sich bekannter Weise Fahrzeugdynamikdaten, die eine Bewegung des Kraftfahrzeugs bezüglich seiner realen Umgebung beschreiben, und Brillendynamikdaten, welche eine Bewegung der Datenbrille bezüglich des Kraftfahrzeugs und/oder der realen Umgebung beschreiben, erfasst. Durch eine Steuervorrichtung wird in Abhängigkeit von den Fahrzeugdynamikdaten und den Brillendynamikdaten eine Perspektive, welche dem Benutzer mittels der Datenbrille angezeigt wird, gesteuert. Die Perspektive ist ein virtueller Blickpunkt oder virtueller Standpunkt sowie eine virtuelle Blickrichtung auf den Anzeigeinhalt der Datenbrille, also beispielsweise auf oder in einen virtuellen Raum. Es handelt sich also um eine Perspektive in eine virtuelle Realität oder einen virtuellen Raum.

Um nun während der Fahrt in dem Kraftfahrzeug, während es aufgrund der Bewegung des Kraftfahrzeugs zu einer Einwirkung einer (translatorischen und/oder rotatorischen) Beschleunigungskraft auf den Benutzer kommt, dies in der angezeigten Perspektive abzubilden oder visuell zu repräsentieren, ist erfindungsgemäß vorgesehen, in der angezeigten Perspektive den Medieninhalt in einem Medienfeld anzuzeigen, also beispielsweise einer virtuellen Kinoleinwand oder einem rechteckigen Feld. Als Medieninhalt kann in der beschriebenen Weise beispielsweise ein Videofilm oder ein Text oder eine Datenpräsentation vorgesehen sein, um nur Beispiele zu nennen. Das Medienfeld wird hierbei (aus der angezeigten Perspektive betrachtet) durch ein Rahmenelement hindurch sichtbar dargestellt, wobei das Rahmenelement das Medienfeld zumindest teilweise, bevorzugt vollständig, umgibt. Ein solches Rahmenelement kann also beispielsweise ein Bilderrahmen sein oder es als ein Fensterrahmen eines virtuellen Cockpits dargestellt sein. Das Rahmenelement ist Teil der graphischen Anzeige, umfasst also z.B. zumindest ein pixelbasiertes Graphikobjekt. Neben dem Rahmenelement und/oder durch einen teiltransparenten Bereich des Rahmenelements hindurch wird eine künstliche Umgebung dargestellt. Die künstliche Umgebung repräsentiert dabei die reale Umgebung in Bezug auf eine relative Bewegung. Die künstliche Umgebung ist ebenfalls Teil der graphischen Anzeige, umfasst also zumindest ein pixelbasiertes Graphikobjekt.

Es wird nun eine Geschwindigkeitskomponente der Fahrzeugdynamikdaten, also eine Angabe zur Fahrgeschwindigkeit des Kraftfahrzeugs bezüglich der realen Umgebung, als eine Bewegung der künstlichen Umgebung bezüglich der Rahmenelements dargestellt. Mit anderen Worte bewegt sich die künstliche Umgebung an dem Rahmenelement vorbei wie die reale Umgebung an dem Kraftfahrzeug. Eine Beschleunigungskomponente der Fahrzeugdynamikdaten, also Beschleunigungsdaten betreffend eine Geschwindigkeitsänderung und/oder Richtungsänderung des Fahrzeugs in der realen Umgebung, wird durch eine Neigung oder Verschiebung des Rahmenelements bezüglich des Medienfelds dargestellt. Eine Beschleunigungskomponente wird also visuell in der angezeigten Perspektive repräsentiert, indem sich das Rahmenfeld bezüglich des Medienfelds in Bezug auf die Relativposition verändert. Dagegen wird eine Bewegungskomponente der Brillendynamikdaten, also Bewegungsdaten, die eine Bewegung des Kopfes des Benutzers relativ zum Kraftfahrzeug und/oder der realen Umgebung beschreiben, als eine Verschiebung der gesamten Perspektive oder dargestellt. Der Benutzer hat hierdurch also weiterhin den Eindruck, dass er sich bezüglich aller dargestellten Elemente frei in einem Raum bewegen kann. Dabei kann auch das Medienfeld unbewegt verharren oder mit dem Kopf mit verschwenkt werden. In letzterem Fall bleibt also der Medieninhalt im Blickfeld des Benutzers, selbst wenn dieser den Kopf zur Seite dreht.

Eine Auswertung mit Testpersonen hat ergeben, dass dieses Verfahren den Vorteil bringt, dass Benutzer Beschleunigungskräfte, die sie während der Fahrt des Kraftfahrzeugs aufgrund ihres Gleichgewichtssinns empfinden, nun visuell zuordnen können, indem das Rahmenelement sich bezüglich des Medienfelds und auch bezüglich der bewegten künstlichen Umgebung neigt. Dies hat in den Tests zu einer vorteilhaften Reduktion der Bewegungskrankheit geführt.

Durch das Verfahren werden hierbei aber nicht einfach nur die Geschwindigkeitskomponente und die Beschleunigungskomponente der Fahrzeugdaten visuell dargestellt, sondern durch die beschriebene Verwendung der Geschwindigkeitskomponente für das Darstellen der Bewegung der künstlichen Umgebung und die Verwendung der Beschleunigungskomponente für das Darstellen der Neigung oder Verschiebung des Rahmenelements bezüglich des Medienfelds ergibt sich eine visuelle Orientierung für den Benutzer, um die von ihm mittels seines Gleichgewichtssinns gespürte Bewegung und/oder Bewegungsveränderung in Einklang mit der angezeigten Perspektive zu bringen, was wichtige Voraussetzung zur Vermeidung der Bewegungskrankheit ist. Das beschriebene Verfahren nutzt also die kognitive Verarbeitung der mittels der Bilddaten oder des Bildstroms angezeigten Perspektive, um im Benutzer einen Einfluss auf dessen Reaktion auf Beschleunigungskräfte zur Reduktion der Bewegungskrankheit zu erhalten.

Bei der Erfindung weist das Medienfeld in der angezeigten Perspektive einen Ausdehnungswinkel in horizontaler und/oder vertikaler Richtung kleiner als 35 % auf. Der Ausdehnungswinkel ist hier gemessen am Sichtwinkel oder Sehwinkel. Die Ausdehnung ist bevorzugt in einem Bereich kleiner als 30 % oder gleich 30 % für die horizontale und vertikale Richtung. Der Ausdehnungswinkel zeigt das Medienfeld also bevorzugt im Bereich des sogenannten fovealen Sehens des Benutzers. Bevorzugt ist hierbei der Ausdehnungswinkel in horizontaler und/oder horizontaler Richtung aber größer als 15°, insbesondere größer als 20° oder gleich 20°. Somit kann der Benutzer das Medienfeld fokussieren und kann somit den Medieninhalt fokussiert wahrnehmen, während er mittels des außer-fovealen Sehens oder des peripheren Sehens Bewegung wahrnimmt, nämlich die Bewegung des Rahmenelements und der künstlichen Umgebung, die ihm visuell die Bewegung des Kraftfahrzeugs in der realen Umgebung vermitteln. Da das Auge für Bewegungswahrnehmung im peripheren Bereich empfindlicher ist als im fovealen Bereich, ergibt sich durch den besagten (auf das foveale Sehen reduzierten) Ausdehnungswinkel des Medienfelds der Vorteil, dass ein besonders großer Bereich des Sichtfelds des Benutzers für die visuelle Vermittlung der Geschwindigkeitskomponente und der Beschleunigungskomponente der Fahrzeugdynamikdaten vermittels wird, da die Bewegung der künstlichen Umgebung und die Neigung des Rahmenelements erkennbar ist.

Bei der Erfindung wird in dem Medienfeld der Medieninhalt teiltransparent mit einer Opazität von mehr als 70 % und weniger als 100 % dargestellt. Hierdurch kann man also durch das Medienfeld anteilig hindurchsehen, wobei die Transparenz entsprechend umgekehrt zur Opazität in einem Bereich größer als 0 % und kleiner als 30 % liegt. Durch das Medienfeld hindurch sieht man überlagert die künstliche Umgebung. Somit wird also auch die Bewegung der künstlichen Umgebung in dem Medienfeld dargestellt. Dies unterstützt in vorteilhafter Weise die Reduktion der Bewegungskrankheit.

Bei der Erfindung weist das Rahmenelement den besagten teiltransparenten Bereich auf. Das Rahmenelement kann vollständig als teiltransparentes Element dargestellt werden oder es kann auch nur ein Teilbereich des Rahmenelements teiltransparent dargestellt werden. Eine Opazität des Rahmenelements in dem teiltransparenten Bereich beträgt dabei höchstens 50 %. Mit anderen Worten ist das Rahmenelement zumindest zu 50 % oder mehr als 50 % transparent dargestellt. Eine teiltransparente Darstellung kann mittels des sogenannten Alpha-Blending bewirkt werden. Durch den teiltransparenten Bereich des Rahmenelements hindurch wird die künstliche Umgebung dargestellt oder sichtbar gemacht. Hierdurch ist vermieden, dass das Rahmenelement dem Benutzer den Blick auf die künstliche Umgebung und/oder Bewegungskomponenten oder Bewegungseindrücke, die von der künstlichen Umgebung vermittelt werden, verstellt oder blockiert.

Die Erfindung umfasst auch Ausführungsformen, durch die sich zusätzliche Vorteile ergeben.

In einer Ausführungsform wird beim Darstellen der Geschwindigkeitskomponente (aus den Fahrdynamikdaten des Kraftfahrzeugs) die Bewegung der künstlichen Umgebung um einen Verstärkungsfaktor größer als 1 schneller dargestellt als die Bewegung der realen Umgebung. Der Verstärkungsfaktor kann in einem Bereich von 2 bis 20 liegen. Beträgt der Verstärkungsfaktor beispielsweise 10, so ergibt sich bei einer Fahrgeschwindigkeit von 10 km/h des Kraftfahrzeugs in der realen Umgebung eine entsprechende relative Bewegung der künstlichen Umgebung bezüglich des Rahmenelements, die 100 km/h entspricht. Der Verstärkungsfaktor kann auch abhängig davon eingestellt sein, welchen absoluten Wert die Fahrgeschwindigkeit des Kraftfahrzeugs aufweist. Ein Verstärkungsfaktor größer als 1 verstärkt die visuelle Wahrnehmung einer Geschwindigkeitsänderung (also einer Beschleunigung oder Abbremsung des Kraftfahrzeugs), so dass hier eine weitere visuelle Repräsentation der empfundenen Beschleunigungskraft gegeben ist, was die Bewegungskrankheit weiter reduzieren kann.

In einer Ausführungsform wird eine in der Beschleunigungskomponente angegebene Längsbeschleunigung des Kraftfahrzeugs (Geschwindigkeitserhöhung oder Geschwindigkeitsreduktion) als ein längskippendes Rahmenelement dargestellt. Das Rahmenelement kippt also bei einem Bremsmanöver nach vorne (das heißt, es neigt sich bezüglich des Medienfelds nach unten oder verschiebt sich nach unten), und bei einer Geschwindigkeitszunahme wird ein Kippen nach hinten angezeigt (das heißt, das Rahmenelement neigt sich bezüglich des Medienfelds nach oben oder verschiebt sich nach oben). Somit korrespondiert die Reaktion oder Veränderung des Rahmenelements mit der Erfahrung des Benutzers, dass ein Kraftfahrzeug eine Nickbewegung ausführt, wenn es zu einer Längsbeschleunigung kommt. Durch diese Übereinstimmung der Erfahrung kann dem Benutzer die visuelle Repräsentation der Beschleunigungskomponente konsistent mit seinem erwarteten visuellen Eindruck präsentiert werden.

In einer Ausführungsform wird eine in der Beschleunigungskomponente angegebene Querbeschleunigung des Kraftfahrzeugs als ein Querkippen oder Drehen des Rahmenelements bezüglich des Medienfelds dargestellt, wobei eine Rechtskurvenfahrt ein Kippen oder Drehen des Rahmenelements nach rechts (also eine Drehung im Uhrzeigersinn) und eine Linkskurvenfahrt ein Kippen oder Drehen des Rahmenelements nach rechts (also eine Drehung gegen den Uhrzeigersinn) angezeigt wird. Dies entspricht einem Rollverhalten oder Neigeverhalten eines Flugzeugs. Es hat sich ergeben, dass die visuelle Vermittlung einer Querbeschleunigung des Kraftfahrzeugs auf diese Weise vom Benutzer als besonders vorteilhaft bei der Reduktion der Bewegungskrankheit empfunden wird.

In einer Ausführungsform wird das Medienfeld von der Beschleunigungskomponente unabhängig dargestellt. Bevorzugt ist das Medienfeld auch unabhängig von der Geschwindigkeitskomponente unabhängig dargestellt. Das Medienfeld bietet als einen visuellen Ankerpunkt oder einen mit einem künstlichen Horizont verankerten Bildinhalt. Hierdurch ergibt sich der Vorteil, dass der Benutzer die Orientierung im Raum beibehält, selbst wenn es zum Beispiel zu einer Kurvenfahrt kommt.

In einer Ausführungsform wird mittels der Beschleunigungskomponente ausschließlich das Rahmenelement in der beschriebenen Weise gekippt. Natürlich wirkt sich die Beschleunigungskomponente indirekt auch auf die Bewegungsgeschwindigkeit der künstlichen Umgebung aus, da die sich mit der Zeit ändert. Die Beschleunigungskomponente selbst, also deren absoluter aktueller Wert, wird aber bevorzugt ausschließlich mittels des Rahmenelement visuell vermittelt oder dargestellt. Mit anderen Worten bleibt insbesondere die künstliche Umgebung unverkippt oder ungeneigt, wenn eine Beschleunigungskraft oder Beschleunigungskomponente wirkt. Hierdurch ergibt sich der Vorteil, dass der Benutzer seine räumliche Orientierung wahrt.

Um das erfindungsgemäße Verfahren in einem Kraftfahrzeug durchzuführen, wird durch die Erfindung eine Steuervorrichtung für eine Datenbrille eines Kraftfahrzeugs bereitgestellt. Die Steuervorrichtung weist eine Prozessoreinrichtung auf, die dazu eingerichtet ist, eine Ausführungsform des erfindungsgemäßen Verfahrens durchzuführen. Die Prozessoreinrichtung kann hierzu zumindest einen Mikroprozessor und/oder zumindest einen Mikrocontroller aufweisen. Die Prozessoreinrichtung kann einen Programmcode aufweisen, der Programminstruktionen umfassen kann, die dazu eingerichtet sind, bei Ausführen durch die Prozessoreinrichtung die Ausführungsform des erfindungsgemäßen Verfahrens auszuführen. Der Programmcode kann in einem Datenspeicher der Prozessoreinrichtung gespeichert sein.

Schließlich gehört zu der Erfindung auch ein Kraftfahrzeug mit einer Datenbrille und einer Ausführungsform der erfindungsgemäßen Steuervorrichtung. Das erfindungsgemäße Kraftfahrzeug ist bevorzugt als Kraftwagen, insbesondere als Personenkraftwagen oder Lastkraftwagen, ausgestaltet.

Die Erfindung umfasst auch die Kombinationen der Merkmale der beschriebenen Ausführungsformen.

Im Folgenden ist ein Ausführungsbeispiel der Erfindung beschrieben. Hierzu zeigt:
- Fig. 1: eine schematische Darstellung einer Ausführungsform des erfindungsgemäßen Kraftfahrzeugs; und
- Fig. 2: eine schematische Darstellung einer Perspektive, wie sie in einer Datenbrille im Kraftfahrzeug von Fig. 1 durch eine Steuervorrichtung dargestellt werden kann.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden. Daher soll die Offenbarung auch andere als die dargestellten Kombinationen der Merkmale der Ausführungsformen umfassen. Des Weiteren sind die beschriebenen Ausführungsformen auch durch weitere der bereits beschriebenen Merkmale der Erfindung ergänzbar.

In den Figuren bezeichnen gleiche Bezugszeichen jeweils funktionsgleiche Elemente.

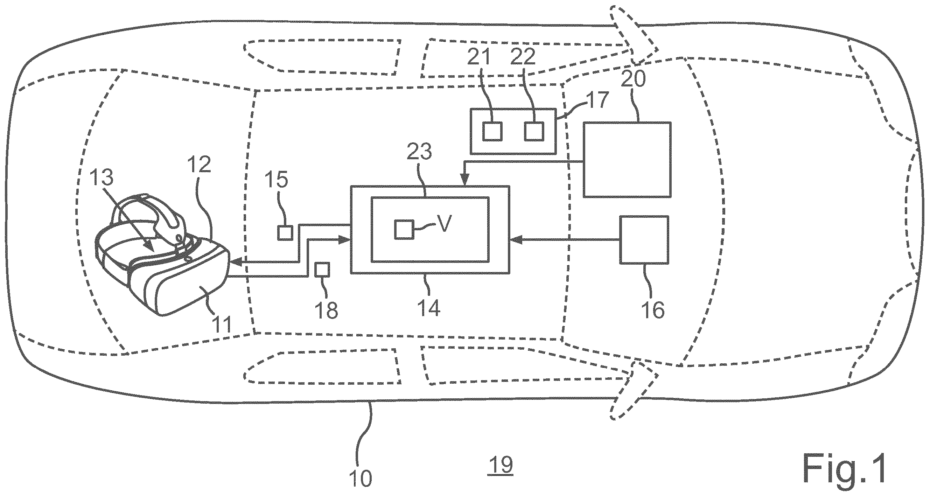

Fig. 1 zeigt ein Kraftfahrzeug 10, bei dem es sich um einen Kraftwagen, insbesondere einen Personenkraftwagen oder Lastkraftwagen, handeln kann. Das Kraftfahrzeug 10 kann für eine Benutzung, beispielsweise durch einen Fahrgast oder (im Falle eines autonomen Fahrmodus) durch einen Fahrer, bereitgestellt sein. Die Datenbrille 11 kann den Augenteil 12 aufweisen, welches beim Tragen der Datenbrille die Augen des Benutzers blickdicht abschließt. In dem Augenteil 12 kann eine Anzeigeeinrichtung 13 integriert sein, mittels welcher dem Benutzer beim Tragen der Datenbrille 11 in dessen Blickfeld ein grafischer Anzeigeinhalt angezeigt werden kann. Eine Steuervorrichtung 14 kann hierzu zum Steuern der Datenbrille 11 und zum Festlegen des Anzeigeinhalts auf der Anzeigeeinrichtung 13 Grafikdaten 15 erzeugen. Somit kann die Steuervorrichtung 14 mittels der Datenbrille 11 insbesondere einen Medieninhalt 16 anzeigen, bei dem es sich beispielsweise um einen Videofilm und/oder Fotografien und/oder Bildinhalte aus dem Internet und/oder einen Text, beispielsweise eine E-Mail oder eine SMS (Short Message Service), und/oder eine Präsentation handeln kann. Während das Kraftfahrzeug 10 fährt, besteht allerdings die Gefahr, dass dem Benutzer schlecht wird, das heißt, dass er Bewegungskrankheit erleidet, weil durch das Augenteil 12 sein Blick das Umfeld des Benutzers nicht erfassen kann, so dass der Benutzer zwar Beschleunigungskräfte mit seinem Gleichgewichtssinn spürt, hierzu aber noch ein visuell korrespondierendes Ereignis sehen muss, damit Bewegungskrankheit reduziert oder verhindert wird.

Hierzu ist in dem Kraftfahrzeug 10 vorgesehen, dass die Steuervorrichtung Fahrzeugdynamikdaten 17 und Brillendynamikdaten 18 nutzt. Die Brillendynamikdaten 18 beschreiben eine Bewegung der Datenbrille 11 bezüglich des Kraftfahrzeugs und/oder einer Umgebung 19 des Kraftfahrzeugs. Die Brillendynamikdaten 18 können beispielsweise mittels zumindest eines Beschleunigungssensors, der in der Datenbrille 11 integriert sein kann, und/oder mittels Funksensoren und/oder optischer Sensoren, die im Kraftfahrzeug 10 bereitgestellt sein können und eine relative Bewegung der Datenbrille 11 erfassen, erzeugt werden.

Die Fahrzeugdynamikdaten 17 können in an sich bekannter Weise aus einer Sensoreinrichtung 20 des Kraftfahrzeugs 10 empfangen werden, beispielsweise über einen Kommunikationsbus, wie beispielsweise einen CAN-Bus (CAN - Controller Area Network). Die Fahrzeugdynamikdaten können insbesondere eine Bewegungskomponente 21 und eine Beschleunigungskomponente 22 beschreiben. Die Bewegungskomponente 21 kann beispielsweise eine Fahrtrichtung und/oder eine Fahrgeschwindigkeit angeben. Die Beschleunigungskomponente 22 kann beispielsweise eine Geschwindigkeitsänderung in Längsrichtung und/oder Querrichtung und/oder um eine senkrechte Rotationsachse (Gierrate) und/oder eine Nickrate (Drehung um eine Querachse) und/oder Rollrate (Drehung um eine Längsachse) beschreiben.

Die Bewegungskomponente kann eine Gierbewegung, das heißt eine Rotation um eine senkrechte Rotationsachse, eine Nickbewegung um eine Querachse und/oder eine Rollbewegung um eine Längsachse des Kraftfahrzeugs 10 beschreiben.

Die Steuervorrichtung 14 kann beispielsweise auf der Grundlage zumindest eines Steuergeräts realisiert sein und für die Durchführung des folgenden Verfahrens eine Prozessoreinrichtung 23 aufweisen.

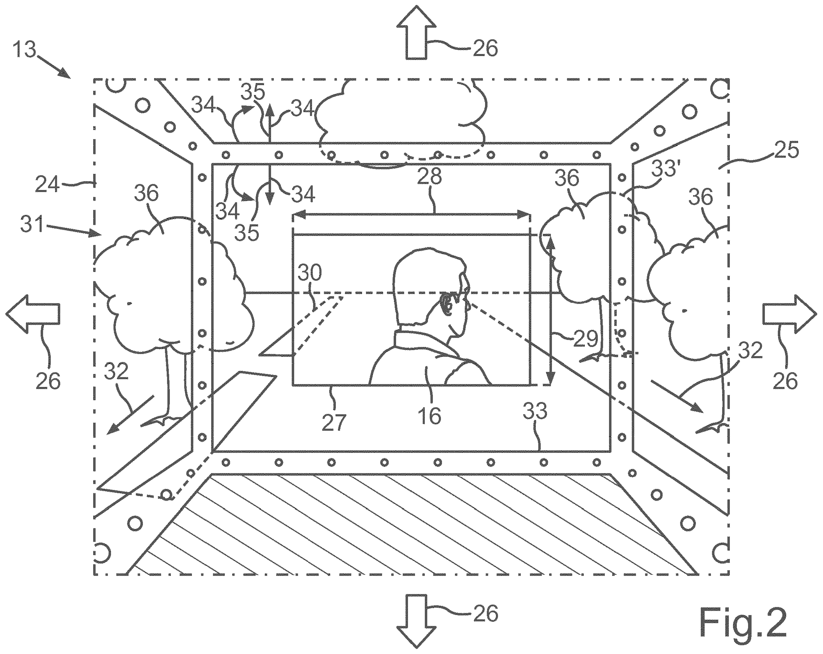

Die Steuervorrichtung 14 kann beispielsweise mittels der Grafikdaten 15 dem Benutzer mittels der Datenbrille 11 den Medieninhalt 16 in der in Fig. 2 dargestellten Weise anzeigen, um zu vermeiden oder zu reduzieren, dass der Benutzer beim Betrachten des Medieninhalts 16 eine Bewegungskrankheit empfindet.

Fig. 2 veranschaulicht eine Perspektive 24, wie sie mittels der Anzeigeeinrichtung 13 dem Benutzer als Blick in einen virtuellen Raum 25 vermittelt werden kann. Eine Verschiebung 26 des Blicks oder eine Blickrichtungsänderung, welche den dargestellten Bildausschnitt oder den dargestellten Blick in den virtuellen Raum 25 zeigt, kann in Abhängigkeit von den Brillendynamikdaten 18 eingestellt werden. Die Verschiebung 26 entspricht einem Kameraschwenk.

In der Perspektive 24 kann der Medieninhalt 16 in einem Medienfeld 27 dargestellt werden, was beispielsweise als ein Rechteck oder eine künstliche Projektionsfläche repräsentiert sein kann. Ein horizontaler Ausdehnungswinkel 28 und/oder ein vertikaler Ausdehnungswinkel 29 kann das Blickfeld des Benutzers bis zu einem Ausdehnungswinkel von 35°, bevorzugt aber weniger, ausfüllen. Der Medieninhalt 16 kann in dem Medienfeld 27 bevorzugt mittels einer Teiltransparenz 30 dargestellt werden, durch welche in dem Medienfeld 27 auch eine künstliche Umgebung 31 sichtbar ist, die außerhalb des Medienfelds 27 weiter dargestellt werden kann. Eine relative Bewegung 32 der künstlichen Umgebung 31 bezüglich des virtuellen Blickpunkts des Benutzers, also bezüglich der Perspektive 24, kann auf der Grundlage der Bewegungskomponente 21 der Fahrzeugdynamikdaten 17 an eine Fahrgeschwindigkeit des Kraftfahrzeugs 10 durch die Umgebung 19 angepasst sein. Es kann hierbei ein Verstärkungsfaktor V größer als 1 betreffend die Bewegung 32 der künstlichen Umgebung im Verhältnis zur Fahrgeschwindigkeit vorgesehen sein. Des Weiteren kann in der Perspektive 24 ein Rahmenelement 33 dargestellt werden, welches das Medienfeld 27 ganz oder teilweise umgeben kann. Es kann beispielsweise mittels des Rahmenelements 33 ein Rahmen eines Fensters eines Cockpits dargestellt sein. Auch für das Rahmenelement 33 kann zumindest für einen Bereich 33' eine Teiltransparenz vorgesehen sein.

Das Rahmenelement 33 kann bezüglich des Medienfelds 27 eine Neigungsbewegung oder Neigung 34 durchführen, wobei ein Kippen 35, das heißt eine Verschiebung nach oben oder unten bezüglich des Medienfelds 27, in Abhängigkeit von der Beschleunigungskomponente 22 der Fahrzeugdynamikdaten eingestellt werden kann, wenn es sich um eine Längsbeschleunigung handelt. Bei einer Querbeschleunigung kann eine Neigung oder ein Verkippen des Rahmenelements 33 bezüglich des Medienfelds 27 und der künstlichen Umgebung 31 vorgesehen sein.

Somit ergibt sich insgesamt eine Kombination mehrerer Ansätze, die bei Testpersonen zu einer Reduktion einer Bewegungskrankheit geführt haben.
1) Die Größe des Medienfelds oder Kino-Screens darf horizontal bevorzugt nur maximal 20° bis 30° betragen (der Winkel bezieht sich auf einen vom Auge aufgespannten Winkel, welcher einen Prozentsatz des Sichtfelds darstellt).
2) Das Medienfeld 27 sollte leicht transparent dargestellt werden (10 bis 20 % Transparenz als Richtwert).
3) Zusätzlich zum Medienfeld 27 sollte ein Cockpit oder allgemein ein Rahmenelement dargestellt werden, in welchem sich der Benutzer befindet. Das Rahmenelement hat bevorzugt folgende Eigenschaften, insbesondere als Cockpit:
   a. Längsbeschleunigungen des Kraftfahrzeugs führen zum Kippen des Cockpits nach vorne und hinten, hierbei stellt ein Bremsmanöver ein Kippen nach vorne dar.
   b. Querbeschleunigungen führen zum Kippen nach links und rechts, wobei Rechtskurven, das heißt eine Beschleunigung nach links, ein Kippen des Rahmenelements nach rechts zur Folge hat (analog der Flugdynamik eines Flugzeugs).
   c. Das Rahmenelement oder Cockpit hat in einem teiltransparenten Bereich 33`, bevorzugt im ganzen Bereich, eine Transparenz von mindestens 50 % (alpha-Wert für das Alpha-Blending). Hierdurch sind vorbeiziehende Objekte 36 ohne Sichteinschränkung wahrnehmbar.
4) Das Medienfeld 27 muss entkoppelt sein vom Kippen des Rahmenelements 33 (z.B. dargestellt als Cockpit).
5) Die Bewegung der Kamera oder die Verschiebung 26 der Perspektive muss entkoppelt sein vom Kippen des Rahmenelements 33 (z.B. dargestellt als Cockpit).

Die angegebenen Werte sind bevorzugte Richtwerte und können vom Fachmann bei der exakten Umsetzung eingestellt oder festgelegt werden.

Durch die beschriebenen Maßnahmen lässt sich die Bewegungskrankheit beim Konsum von Medieninhalten (insbesondere klassischen Medien, wie zum Beispiel Filmen, Serien, Text- und/oder Präsentationen) reduzieren.

## Patentansprüche

1. Verfahren zum Reduzieren von Bewegungskrankheit eines Benutzers beim Betrachten eines Medieninhalts (16) mittels einer Datenbrille (11) während einer Fahrt in einem Kraftfahrzeug (10), wobei durch eine Steuervorrichtung (14) Fahrzeugdynamikdaten (17), die eine Bewegung des Kraftfahrzeugs (10) bezüglich seiner realen Umgebung (19) beschreiben, aus einer Sensoreinrichtung (20) des Kraftfahrzeugs (10) empfangen werden und Brillendynamikdaten (18), welche eine Bewegung der Datenbrille (11) bezüglich des Kraftfahrzeugs (10) und/oder der realen Umgebung (19) beschreiben und damit eine Bewegung eines Kopfes des Benutzers relativ zum Kraftfahrzeug und/oder der realen Umgebung beschreiben, mittels zumindest eines Beschleunigungssensors, der in der Datenbrille (11) integriert ist, und/oder mittels Funksensoren und/oder optischer Sensoren, die im Kraftfahrzeug (10) bereitgestellt sind und eine relative Bewegung der Datenbrille (11) erfassen, erfasst werden und in Abhängigkeit von den Fahrzeugdynamikdaten (17) und den Brillendynamikdaten (18) eine Perspektive (24), welche dem Benutzer mittels der Datenbrille (11) angezeigt wird, gesteuert wird,
**dadurch gekennzeichnet, dass**
in der angezeigten Perspektive (24) der Medieninhalt (16) in einem Medienfeld (27) angezeigt wird und das Medienfeld (27) durch ein Rahmenelement (33) hindurch sichtbar dargestellt wird, wobei das Rahmenelement (33) das Medienfeld (27) zumindest teilweise umgibt, und neben dem Rahmenelement (33) eine künstliche Umgebung (25) dargestellt wird, wobei
eine Geschwindigkeitskomponente (21) der Fahrzeugdynamikdaten (17) als eine Bewegung (32) der künstlichen Umgebung (25) bezüglich des Rahmenelements (33) dargestellt wird und eine Beschleunigungskomponente (22) der Fahrzeugdynamikdaten (17) durch eine Neigung (34) des Rahmenelements (33) bezüglich des Medienfelds (27) dargestellt wird und eine Bewegungskomponente der Brillendynamikdaten (18) als eine Verschiebung (26) der gesamten Perspektive (24) ohne das Medienfeld (27) dargestellt wird, indem das Medienfeld (27) mit dem Kopf mit verschwenkt wird, und wobei die Größe des Medienfelds horizontal weniger als 35° beträgt, wobei der Winkel sich auf einen vom Auge aufgespannten Winkel bezieht, und
in Abhängigkeit von der Beschleunigungskomponente (22) ausschließlich das Rahmenelement (33) gekippt wird, wobei
das Rahmenelement (33)
• vollständig als teiltransparentes Element dargestellt wird oder
• nur ein Teilbereich des Rahmenelements (33) teiltransparent dargestellt wird,
und durch den jeweiligen teiltransparenten Bereich (33') des Rahmenelements (33) hindurch die künstliche Umgebung (25) dargestellt wird, wobei eine Opazität des Rahmenelements (33) in dem teiltransparenten Bereich (33') höchstens 50% beträgt, und wobei in dem Medienfeld (27) der Medieninhalt (16) teiltransparent mit einer Opazität von mehr als 70% und weniger als 100% dargestellt und der künstlichen Umgebung (25) überlagert wird.

2. Verfahren nach Anspruch 1, wobei beim Darstellen der Geschwindigkeitskomponente (21) die Bewegung (32) der künstlichen Umgebung (25) um einen Verstärkungsfaktor (V) größer als 1 schneller dargestellt wird als die Bewegung (32) der realen Umgebung (19).

3. Verfahren nach einem der vorhergehenden Ansprüche, wobei eine in der Beschleunigungskomponente (22) angegebene Längsbeschleunigung des Kraftfahrzeugs (10) als ein Längskippen des Rahmenelements dargestellt wird, wobei ein Bremsmanöver als ein Kippen nach vorne und eine Geschwindigkeitszunahme als ein Kippen nach hinten angezeigt wird.

4. Verfahren nach einem der vorhergehenden Ansprüche, wobei eine in der Beschleunigungskomponente (22) angegebene Querbeschleunigung des Kraftfahrzeugs (10) als ein Drehen des Rahmenelements (33) dargestellt wird, wobei eine Rechtskurvenfahrt als ein Drehen des Rahmenelements (33) nach rechts und eine Linkskurvenfahrt als ein Drehen des Rahmenelements (33) nach links angezeigt wird.

5. Verfahren nach einem der vorhergehenden Ansprüche, wobei das Medienfeld (27) von der Beschleunigungskomponente (22) unabhängig dargestellt wird.

6. Steuervorrichtung (14) für eine Datenbrille (11) eines Kraftfahrzeugs (10), wobei die Steuervorrichtung (14) eine Prozessoreinrichtung (23) aufweist, die dazu eingerichtet ist, ein Verfahren nach einem der vorhergehenden Ansprüche durchzuführen, und dazu eingerichtet ist Fahrzeugdynamikdaten (17), die eine Bewegung des Kraftfahrzeugs (10) bezüglich seiner realen Umgebung (19) beschreiben, aus einer Sensoreinrichtung (20) des Kraftfahrzeugs (10) zu empfangen und Brillendynamikdaten (18), welche eine Bewegung der Datenbrille (11) bezüglich des Kraftfahrzeugs (10) und/oder der realen Umgebung (19) beschreiben und damit eine Bewegung eines Kopfes des Benutzers relativ zum Kraftfahrzeug und/oder der realen Umgebung beschreiben, mittels zumindest eines Beschleunigungssensors, der in der Datenbrille (11) integriert ist, und/oder mittels Funksensoren und/oder optischer Sensoren, die im Kraftfahrzeug (10) bereitgestellt sind und eine relative Bewegung der Datenbrille (11) erfassen, zu erfassen.

## Claims

1. A method for reducing motion sickness of a user in viewing a media content (16) by means of data goggles (11) during a travel in a motor vehicle (10), wherein, by a control device (14), vehicle dynamic data (17), which describes a motion of the motor vehicle (10) with respect to its real environment (19), is received from a sensor device (20) of the motor vehicle (10) and goggle dynamic data (18), which describes a motion of the data goggles (11) with respect to the motor vehicle (10) and/or to the real environment (19) and thereby describes a motion of a head of the user in relation to the motor vehicle and/or to the real environment, is captured by means of at least one acceleration sensor integrated in the data goggles (11) and/or by means of radio sensors and/or optical sensors provided in the motor vehicle (10) and capturing a relative motion of the data goggles (11), and a perspective (24), which is displayed to the user by means of the data goggles (11), is controlled depending on the vehicle dynamic data (17) and the goggle dynamic data (18),
**characterized in that**
the media content (16) is displayed in a media field (27) in the displayed perspective (24) and the media field (27) is represented visible through a frame element (33), wherein the frame element (33) at least partially surrounds the media field (27), and an artificial environment (25) is represented next to the frame element (33), wherein
a speed component (21) of the vehicle dynamic data (17) is represented as a motion (32) of the artificial environment (25) with respect to the frame element (33), and an acceleration component (22) of the vehicle dynamic data (17) is represented by an inclination (34) of the frame element (33) with respect to the media field (27), and a motion component of the goggle dynamic data (18) is represented as a displacement (26) of the entire perspective (24) without the media field (27) **in that** the media field (27) is pivoted together with the head, and wherein the size of the media field is horizontally less than 35°, wherein the angle relates to an angle spanned by the eye, and
depending on the acceleration component (22), exclusively the frame element (33) is tilted, wherein
the frame element (33)
• is completely represented as a partially transparent element, or
• only a partial area of the frame element (33) is partially transparently represented,
and the artificial environment (25) is represented through the respective partially transparent area (33') of the frame element (33),
wherein an opacity of the frame element (33) in the partially transparent area (33') is at most 50 %, and wherein the media content (16) is partially transparently represented with an opacity of more than 70 % and less than 100 % and overlaid on the artificial environment (25) in the media field (27).

2. The method according to claim 1, wherein in representing the speed component (21), the motion (32) of the artificial environment (25) is represented faster by a gain factor (V) of greater than 1 than the motion (32) of the real environment (19).

3. The method according to any one of the preceding claims, wherein a longitudinal acceleration of the motor vehicle (10) indicated in the acceleration component (22) is represented as a longitudinal tilt of the frame element, wherein a braking maneuver is displayed as a forward tilt and a speed increase is displayed as a rearward tilt.

4. The method according to any one of the preceding claims, wherein a lateral acceleration of the motor vehicle (10) indicated in the acceleration component (22) is represented as a rotation of the frame element (33), wherein right cornering is displayed as a rotation of the frame element (33) to the right and left cornering is displayed as a rotation of the frame element (33) to the left.

5. The method according to any one of the preceding claims, wherein the media field (27) is represented independently of the acceleration component (22).

6. A control device (14) for data goggles (11) of a motor vehicle (10), wherein the control device (14) comprises a processor device (23), which is configured to perform a method according to any one of the preceding claims, and is configured to receive vehicle dynamic data (17), which describes a motion of the motor vehicle (10) with respect to its real environment (19), from a sensor device (20) of the motor vehicle (10) and to capture goggle dynamic data (18), which describes a motion of the data goggles (11) with respect to the motor vehicle (10) and/or to the real environment (19) and thereby describes a motion of a head of the user in relation to the motor vehicle and/or to the real environment, by means of at least one acceleration sensor integrated in the data goggles (11) and/or by means of radio sensors and/or optical sensors, which are provided in the motor vehicle (10) and capture a relative motion of the data goggles (11).

## Revendications

1. Procédé destiné à réduire le mal des transports d'un utilisateur observant un contenu multimédia (16) au moyen de lunettes intelligentes (11) pendant un trajet dans un véhicule à moteur (10), dans lequel des données dynamiques de véhicule (17) qui décrivent un mouvement du véhicule à moteur (10) par rapport à son environnement réel (19), provenant d'un dispositif de capteurs (20) du véhicule à moteur (10), sont reçues par un dispositif de commande (14), et des données dynamiques de lunettes (18) qui décrivent un mouvement des lunettes intelligentes (11) par rapport au véhicule à moteur (10) et/ou à l'environnement réel (19) et qui décrivent ainsi un mouvement d'une tête de l'utilisateur par rapport au véhicule à moteur et/ou à l'environnement réel, sont détectées au moyen d'au moins un capteur d'accélération qui est intégré dans les lunettes intelligentes (11) et/ou au moyen de capteurs radio et/ou de capteurs optiques qui sont fournis dans le véhicule à moteur (10) et qui détectent un mouvement relatif des lunettes intelligentes (11), et une perspective (24) affichée à l'utilisateur au moyen des lunettes intelligentes (11) est commandée en fonction des données dynamiques de véhicule (10) et des données dynamiques de lunettes (18),
**caractérisé en ce que**
dans la perspective (24) affichée, le contenu multimédia (16) est affiché dans un champ multimédia (27) et le champ multimédia (27) est affiché de manière visible à travers un élément de cadre (33), l'élément de cadre (33) entourant au moins partiellement le champ multimédia (27) et un environnement artificiel (25) étant affiché à côté de l'élément de cadre (33),
une composante de vitesse (21) des données dynamiques de véhicule (17) étant représentée sous la forme d'un mouvement (32) de l'environnement artificiel (25) par rapport à l'élément de cadre (33), et une composante d'accélération (22) des données dynamiques de véhicule (17) étant représentée par une inclinaison (34) de l'élément de cadre (33) par rapport au champ multimédia (27), et une composante de mouvement des données dynamiques de lunettes (18) étant représentée sous la forme d'un déplacement (26) de la perspective complète (24) sans le champ multimédia (27) en faisant pivoter le champ multimédia (27) avec la tête, et la taille du champ multimédia à l'horizontale étant inférieure à 35°, l'angle se rapportant à l'angle défini par l'oeil, et
l'élément de cadre (33) étant basculé exclusivement en fonction de la composante d'accélération (22),
• l'élément de cadre (33) étant entièrement représenté sous la forme d'un élément partiellement transparent ou
• seule une zone partielle de l'élément de cadre (33) étant représentée de manière partiellement transparente,
et l'environnement artificiel (25) étant affiché à travers la zone partiellement transparente (33') respective de l'élément de cadre (33), une opacité de l'élément de cadre (33) dans la zone partiellement transparente (33') étant d'au plus 50 %, et le contenu multimédia (16) dans le champ multimédia (27) étant affiché de manière partiellement transparente avec une opacité supérieure à 70 % et inférieure à 100 %, et étant superposé à l'environnement artificiel (25).

2. Procédé selon la revendication 1, dans lequel lors de l'affichage de la composante de vitesse (21), le mouvement (32) de l'environnement artificiel (25) est représenté plus rapidement, d'un facteur d'amplification (V) supérieur à 1, que le déplacement (32) de l'environnement réel (19).

3. Procédé selon l'une des revendications précédentes, dans lequel une accélération longitudinale du véhicule à moteur (10) spécifiée dans la composante d'accélération (22) est représentée sous la forme d'un basculement longitudinal de l'élément de cadre, une manoeuvre de freinage étant indiquée par un basculement vers l'avant et une augmentation de vitesse étant indiquée par un basculement vers l'arrière.

4. Procédé selon l'une des revendications précédentes, dans lequel une accélération latérale du véhicule à moteur (10) spécifiée dans la composante d'accélération (22) est représentée sous la forme d'une rotation de l'élément de cadre (33), un virage à droite étant affiché sous la forme d'une rotation de l'élément de cadre (33) vers la droite et un virage à gauche étant affiché sous la forme d'une rotation de l'élément de cadre (33) vers la gauche.

5. Procédé selon l'une des revendications précédentes, dans lequel le champ multimédia (27) est représenté indépendamment de la composante d'accélération (22).

6. Dispositif de commande (14) pour des lunettes intelligentes (11) d'un véhicule à moteur (10), le dispositif de commande (14) comportant un dispositif à processeur (23) configuré pour mettre en oeuvre un procédé selon l'une des revendications précédentes, et configuré pour recevoir des données dynamiques de véhicule (17) qui décrivent un mouvement du véhicule à moteur (10) par rapport à son environnement réel (19), provenant d'un dispositif de capteurs (20) du véhicule à moteur (10), et pour détecter des données dynamiques de lunettes (18) qui décrivent un mouvement des lunettes intelligentes (11) par rapport au véhicule à moteur (10) et/ou à l'environnement réel (19) et qui décrivent ainsi un mouvement d'une tête de l'utilisateur par rapport au véhicule à moteur et/ou à l'environnement réel, au moyen d'au moins un capteur d'accélération qui est intégré dans les lunettes intelligentes (11) et/ou au moyen de capteurs radio et/ou de capteurs optiques qui sont fournis dans le véhicule à moteur (10) et qui détectent un mouvement relatif des lunettes intelligentes (11).
